# FASCICULE DE BREVET EUROPEEN

(11) **EP 1 641 461 B1**
(45) Date de publication et mention de la délivrance du brevet: **29.09.2010**
(21) Numéro de dépôt: 04767475.9
(22) Date de dépôt: 25.06.2004
(51) Int. Cl.: A61K 31/551, A61K 31/513, A61K 31/554, A61K 31/55, A61K 31/137, A61K 31/4412, A61K 31/496, A61P 25/24, A61P 25/18, A61K 31/40, A61K 31/4025

(54) **ASSOCIATION D'UN ANTAGONISTE OU AGONISTE INVERSE DU RECEPTEUR H3 DE L'HISTAMINE AVEC UN ANTI-PSYCHOTIQUE OU UN ANTIDEPRESSEUR ET SON UTILISATION POUR PREPARER UN MEDICAMENT PREVENANT DES EFFETS INDESIRABLES DE MEDICAMENTS PSYCHOTROPES**
KOMBINATIONSPRODUKT MIT EINEM ANTAGONISTEN ODER INVERSEN AGONISTEN VON HISTAMINE-REZEPTOR H<SB>3</SB> UND EINEM ANTIPSYCHOTISCHEN ODER ANTIDEPRESSIVEN MITTEL, UND IHRE VERWENDUNG ZUR HERSTELLUNG EINES MEDIKAMENTS ZUR VERHINDERUNG DER NACHTEILIGEN WIRKUNGEN VON PSYCHOTROPIKA
COMBINATION PRODUCT COMPRISING AN ANTAGONIST OR INVERSE AGONIST OF HISTAMINE RECEPTOR H<SB>3</SB> AND AN ANTIPSYCHOTIC OR ANTIDEPRESSANT AGENT, AND USE THEREOF FOR THE PREPARATION OF A MEDICAMENT THAT PREVENTS THE ADVERSE EFFECTS OF PSYCHOTROPIC DRUGS

(30) Priorité: 27.06.2003 FR 0307836
(43) Date de publication de la demande: 05.04.2006
(73) Titulaire: BIOPROJET, 75003 Paris (FR)
(72) Inventeur: SCHWARTZ, Jean-Charles, F-75014 Paris (FR); LECOMTE, Jeanne-Marie, F-75003 Paris (FR)
(74) Mandataire: Colombet, Alain André
(86) Numéro de dépôt international: PCT/FR2004/001628
(87) Numéro de publication internationale: WO 2005/000315

(56) Documents cités:
- EP-A- 0 982 300
- EP-A- 1 428 820
- US-A1- 2002 065 278
- US-A1- 2003 096 808
- US-B1- 6 248 765
- VOHORA D ET AL: "Histamine and selective H3-receptor ligands: a possible role in the mechanism and management of epilepsy." PHARMACOLOGY, BIOCHEMISTRY, AND BEHAVIOR. UNITED STATES APR 2001, vol. 68, no. 4, avril 2001 (2001-04), pages 735-741, XP002269925 ISSN: 0091-3057
- STARK H ET AL: "Developments of Histamine H3-receptor Antagonists" DRUGS OF THE FUTURE, BARCELONA, ES, vol. 21, no. 5, 1996, pages 507-520, XP002084872 ISSN: 0377-8282
- MONTI J M ET AL: "Effects of selective activation or blockade of the histamine H3 receptor on sleep and wakefulness." EUROPEAN JOURNAL OF PHARMACOLOGY. NETHERLANDS 3 DEC 1991, vol. 205, no. 3, 3 décembre 1991 (1991-12-03), pages 283-287, XP002269926 ISSN: 0014-2999
- MIYAZAKI S ET AL: "Effects of thioperamide, a histamine H3-receptor antagonist, on a scopolamine-induced learning deficit using an elevated plus-maze test in mice." LIFE SCIENCES. ENGLAND 1995, vol. 57, no. 23, 1995, pages 2137-2144, XP002269927 ISSN: 0024-3205
- GANELLIN C R ET AL: "Synthesis of potent non-imidazole histamine H3-receptor antagonists" ARCHIV DER PHARMAZIE 1998 GERMANY, vol. 331, no. 12, 1998, pages 395-404, XP002307663 ISSN: 0365-6233
- LIEDTKE SUSANNA ET AL: "Replacement of imidazole by a piperidine moiety differentially affects the potency of histamine H3-receptor antagonists." NAUNYN-SCHMIEDEBERG'S ARCHIVES OF PHARMACOLOGY. GERMANY JAN 2003, vol. 367, no. 1, janvier 2003 (2003-01), pages 43-50, XP002269928 ISSN: 0028-1298
- MEIER GALINA ET AL: "Influence of imidazole replacement in different structural classes of histamine H3-receptor antagonists" EUROPEAN JOURNAL OF PHARMACEUTICAL SCIENCES, vol. 13, no. 3, juin 2001 (2001-06), pages 249-259, XP002269929 ISSN: 0928-0987
- SHADBOLT R S ET AL: "Some Aryloxyalkylamines, N-Arylethylenediamines and Related Compounds as Anorectic Agents" JOURNAL OF MEDICINAL CHEMISTRY, AMERICAN CHEMICAL SOCIETY. WASHINGTON, US, vol. 14, no. 9, 1971, pages 836-842, XP002084867 ISSN: 0022-2623

## Description

La présente invention a trait à un nouveau médicament constitué par une nouvelle association à usage antipsychotique ou plus généralement psychotrope.

Un certain nombre d'agents psychotropes administrés de manière chronique présentent l'inconvénient d'induire chez les malades une prise de poids exagérée qui constitue un effet extrêmement gênant.

Ceci est particulièrement vrai pour les antipsychotiques de deuxième génération, les agents les plus employés actuellement dans la schizophrénie (Blackburn G.L., J. Clin. Psych. 2000, 61, 36 ; Bobes J. et al., Schizophrenia Res. 2003, 62, 77) mais concerne aussi un certain nombre d'antidépresseurs et autres agents psychotropes (Ackerman S. et Nolom L.J., CNS Drugs 1998, 9, 135).

C'est ainsi que le traitement continu par des agents antipsychotiques tels que l'olanzapine, la rispéridone, la clozapine, la quiétiapine ou antidépresseurs tels que la mirtazapine, l'amitryptiline ou la paroxétine induisent des prises de poids mensuelles de 1 à 2,5 kg et des prises de poids totales pouvant dépasser 4 kg (Bobes et al., 2003). Cet effet indésirable se produit jusqu'à près de 50 % des malades traités chez lesquels il se révèle extrêmement gênant, particulièrement chez les femmes. Il conduit souvent à réduire le bénéfice du traitement aux yeux du malade qui en réduit les doses ou même l'abandonne, ce qui amène à des rechutes de plus en plus difficiles à traiter. Par ailleurs, il est établi que des prises de poids de cette amplitude sont de nature à augmenter significativement les risques de diabète de type 2, d'affections cardiovasculaires et de cancer. Plusieurs tentatives pour prévenir cet effet indésirable par l'association des antipsychotiques à des agents tels que la fluoxétine (Bustillo J.R. et al. Neuropsychopharmacol. 2003, 28, 527), la sibutramine (Heiman et al., World J. Biol. Psycho. 2001, 2, 2515), l'amantadine (Baptista et al., Pharmacopsychiatry 1997, 30, 43) ou le tamoxifène (Baptista et al., Pharmacopsychiatry 1997, 57, 215) n'ont pas été suffisamment probantes pour être suivies d'application thérapeutique.

Le mécanisme de survenue de cet effet indésirable reste discuté, car la plupart des agents psychotropes interagissent avec plusieurs récepteurs cérébraux. On a notamment invoqué à cet égard le blocage des récepteurs 5HT_{2A}, 5HT_{2c}, α-adrénergiques ou histaminergiques H₁.

La demande EP 0 982 300 ainsi que la demande US 2002/065278 décrivent des antagonistes ou agonistes inverses du récepteur H3 de l'histamine. Néanmoins, ces documents ne décrivent ni ne suggèrent les associations de ces composés avec un agent antipsychotique ou antidépresseur antagoniste du récepteur H1 de l'histamine.

Liedtke et al., Naunyn Schmiedeberg's Archives of Pharmacology, 367, 1, 2003, 43-50, Meier Galina et al., European Journal of Pharmaceutical Sciences, 13, 2001, 249-259 et Shadbolt et al., Journal of Medicinal Chemistry, American Chemical Society, 14, 9, 1971, 836-842 décrivent divers antagonistes du récepteur H3 de l'histamine.

La présente invention a pour objectif de remédier à ces inconvénients et de permettre des traitements antipsychotiques ou plus généralement psychotropes par des antipsychotiques neuroleptiques dits de seconde génération, en évitant ou en limitant la prise de poids liée au traitement et en prévenant ou retardant l'apparition de complications associées et notamment les risques de diabètes de type 2, d'affections cardiovasculaires et/ou de maladies néoplasiques.

Un autre objectif de l'invention est de fournir un tel moyen de traitement sensiblement exempt d'autres effets secondaires.

Un autre objectif encore de l'invention est, dans certains cas, d'améliorer l'efficacité du traitement antipsychotique.

La présente invention a pour objet un nouveau médicament comprenant, dans un véhicule pharmaceutiquement acceptable, un anti-psychotique ou un antidépresseur (A), présentant seul un effet indésirable de prise de poids corporel ou de sédation du principalement à un effet antagoniste de l'histamine (H₁), choisi parmi les agents antipsychotiques tel que l'olanzapine, la rispéridone, la clozapine, laquiétiapine ou antidépresseurs tels que la mirtazapine l'aripiprazole et plus préférentiellement l'olanzapine et un antagoniste et/ou agoniste inverse (B) du récepteur H₃ de l'histamine, ledit antipsychotique ou antidépresseur étant présent dans le médicament en une quantité thérapeutiquement efficace pour l'effet antipsychotique ou antidépresseur recherché, et ledit antagoniste et/ou agoniste inverse du récepteur H₃ de l'histamine étant présent en une quantité thérapeutiquement efficace pour assurer l'un au moins des trois effets suivants : suppression ou au moins limitation de l'effet indésirable de prise de poids, suppression ou limitation de l'effet indésirable sur la vigilance, augmentation de l'effet pro-cognitif du traitement.

De préférence, les doses d'antipsychotique et/ou d'antidépresseur sont égales ou similaires à celles des médicaments antipsychotiques ou antidépresseurs correspondants déjà utilisés.

Les antagoniste/antagoniste inverse (B) de l'histamine sur le récepteur H₃ répondent à la formule (II) de la revendication 1. On peut notamment utiliser, conformément à la présente invention, un dérivé de l'imidazole, de nombreux dérivés ayant été décrits, par exemple dans les documents énumérés ci-après.

Des composés (B) ont été décrits dans la demande PCT/EP99/01744 (US SN09/622199, et demande divisionnaire 10/856,838).

De préférence, dans la formule (II), R1 et R2 pris ensemble avec l'atome d'azote auquel ils sont fixés, forment un cycle azoté saturé. 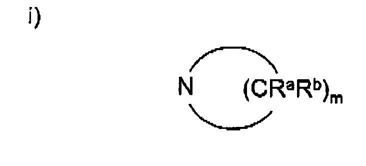 avec m de 2 à 8 , de préférence 5 ; et R^{a-b} représentent indépendamment un atome d'hydrogène ou un groupe alkyle en C1-C6, de préférence un atome d'hydrogène;

De préférence, dans la formule (II), la chaîne A" est choisie parmi les chaînes hydrocarbonées saturées ou insaturées, linéaires ou ramifiées, contenant 1 à 6 atomes de carbone , de préférence, les chaînes alkyles en C1-C6;

De préférence, dans la formule (II), X" est -O- ;

De préférence, dans la formule (II), B" est choisi parmi un groupe aryle, une chaîne alkylène linéaire -(CH₂)ₙ-, n étant de à 5, ou une chaîne alkylène ramifiée contenant 2 à 8 atomes de carbone, la chaîne alkylène étant éventuellement interrompue par un ou plusieurs atomes d'oxygène ou de soufre ; de préférence une chaîne alkylène linéaire -(CH₂)ₙ-, n étant de 1 à 5 ;

De préférence dans la formule générale (II), Y" représente un groupe phényle non substitué ou mono ou polysubstitué par un ou plusieurs substituants identiques ou différents choisis parmi les atomes d'halogène, OCF₃, -CN, -alkyle, -aryle, -alkyleCOAlkyle, -COOAlkyle, -COalkyle, -COaryle, -COaralkyle, -COcycloalkyle, -OH, -alkyle(OH), -alkyle(Oalkyle), -Oaryle, -NHCOAlkyle, -Oalkyle, -C(alkyle)=N-OH, -C(alkyle)=N-O(alkyle), -NO2, -NH2, CHO, CF₃, SO₂N(alkyle)₂ tel que SO₂N(CH₃)₂,
ou un noyau phényle fusionné à un hétérocycle comprenant un hétéroatome d'azote ou à un carbocycle, ledit noyau fusionné pouvant être éventuellement substitué par les substituants précités ; de préférence, Y" représente une groupe phényle éventuellement substitué par un atome d'halogène.

Parmi les composés de formule (II), on préfère ceux de formule (IIa) 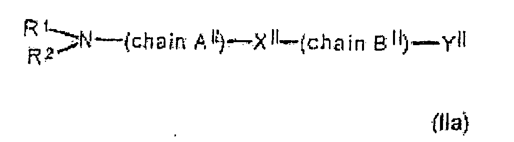 dans laquelle:
R¹ et R², identiques ou différents, représente chacun indépendamment
   - un cycle azoté saturé 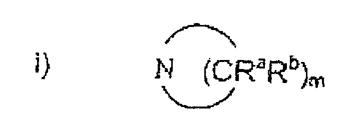 avec m compris entre 2 to 8,
R^{a-b} étant choisis indépendamment parmi un atome d'hydrogène ou un radical alkyle en C1-C4, et
   ii) la chaîne A^{II} est choisie parmi un groupe alkylelinéaire -(CH₂)_{nII}- pour lequel n_{II} est 3 ;
   iii) le groupe X" est -O- ;
   iv) la chaîne B^{II} est un groupe alkyle linéaire comprenant 3 atomes de carbone; et
   v) le group Y^{II} représente un groupe phényle, non-substitué ou mono- ou polysubstitué avec un ou plusieurs substituants identiques ou différents choisis parmi les atomes d'halogène, OCF₃, CHO, CF₃, SO₂N(alkyl)₂ tel que SO₂N(CH₃)₂, NO₂, S(aryl), SCH₂(phenyl), -alkyleCOAlkyle, -COOAlkyle, -COalkyle, -COaryle, -COaralkyle, -COcycloalkyle, -alkyle(OH), -alkyle(Oalkyle), -Oaryle, -NHCOAlkyle, -Oalkyle, -C(alkyle)=N-OH , -C(alkyle)=N-O(alkyle), -NO2, -NH2, un alkène linéaire ou ramifié, un alkyne linéaire ou ramifié éventuellement substitué avec un radical trialkylsilyle, -O(alkyl), -O(aryl), -CH₂CN, une cétone, un aldéhyde, une sulphone, un acétal, un alcool, un alkyl en C1-C4, -CH=CH-CHO, -C(alkyl)=N-OH, -C(alkyl)=N-O(alkyl) et autres dérivés cétoniques, -CH=NOH, -CH=NO(alkyl), et autres dérivés aldéhydes, -C(alkyl)=NH-NH-CONH₂, O-phényl or -OCH₂(phényl), -C(cycloalkyl)=NOH, -C(cycloalkyl)=N-O(alkyl).

De préférence dans la formule (IIa), m est égal à 4 or 5.

De préférence dans la formule (IIa), -NR¹R² est choisi parmi le groupe pipéridyle ou pyrrolidinyle.

De préférence dans la formule (IIa), R^{a} est une atome d'hydrogène.

De préférence dans la formule (IIa), le cycle azoté saturé est mono- ou di-substitué.

De préférence dans la formule (IIa), le cycle azoté saturé est mono-substitué avec un groupe alkyle, de préférence méthyle.

De préférence, dans la formule (IIa), le(s)dit(s) substituant(s) est(sont) en position beta par rapport à l'atome d'azote.

De préférence, dans la formule (IIa), Y^{II} représente un groupe phényle au moins monosubstitué par un groupe cétonique pouvant inclure une cétone à chaine aliphatique en C1-C8 et éventuellement substitué par un groupe hydroxyle, un groupe cycloalkylcétone, araylalkylcétone ou arylalkenylcétone, dans lequel le groupe aryle est éventuellement substitué, ou un groupe hétéroarylcétone.

De préférence dans la formule (IIa), Y^{II} est un phényle au moins mono-substitué par -CHO, une cétone, un aldéhyde, -CH=CH-CHO, -C(alkyl)=N-OH, -C(alkyl)=N-O(alkyl) et autres dérivés cétoniques, -CH=N-OH, -CH=NO(alkyl) et autres dérivés aldéhyde, -C(cycloalkyl)=NOH, -C(cycloalkyl)=N-O(alkyl). De préférence dans la formule (IIa), Y" est un phényle substitué par un atome d'halogéne.

De préférence, le composé de formule (IIa) est choisi parmi :
- 3-Phenylpropyl 3-piperidinopropyl ether
- 3-(4-Chlorophenyl)propyl 3-piperidinopropyl ether
- 3-Phenylpropyl 3-(4-methylpiperidino)propyl ether
- 3-Phenylpropyl 3-(3,5-cis-dimethylpiperidino)propyl ether
- 3-Phenylpropyl 3-(3,5-trans-dimethylpiperidino)propyl ether
- 3-Phenylpropyl 3-(3-methylpiperidino)propyl ether
- 3-Phenylpropyl 3-pyrrolidinopropyl ether
- 3-(4-Chlorophenyl)propyl 3-(4-methylpiperidino)propyl ether
- 3-(4-Chlorophenyl) propyl 3-(3,5-cis-dimethyl piperidino)propyl ether
- 3-(4-Chlorophenyl) propyl 3-(3,5-trans-dimethyl piperidino)propyl ether.

De façon encore plus préférentielle, le composé de formule (IIa) est le 3-(4-chlorophenyl)propyl-3-piperidinopropylether, ou ses sels pharmaceutiquement acceptables, hydrates, ou sels hydratés, ou les structures crystallines polymorphiques de ces composés ou leurs isomères optiques, racémates, diastéréoisomères ou énantiomères.

De préférence, les composés de formule (IIa) se présentent sous la forme de sels pharmaceutiquement acceptables et lesdits sels sont choisis parmi le chlorhydrate, le bromohydrate, l'hydrogènomaléate ou l'hydrogènooxalate.

Les formules et définitions de composés selon l'invention indiquées ci-dessus couvrent les composés sous forme libre ainsi que leurs sels pharmaceutiquement acceptables, hydrates, ou sels hydratés, ou les structures crystallines polymorphiques de ces composés ou leurs isomères optiques, racémates, diastéréoisomères ou énantiomères.

Plus précisément, les composés peuvent être également présent sous forme de ses sels pharmaceutiquement acceptables ou hydrates ou sels hydratés ou structures cristallines polymorphiques ou leurs isomères optiques, racémates, diastéréoisomères ou énantiomères, ayant la fonction d'un ligand antagoniste sur les récepteurs de l'histamine H₃.

Les sels préférés incluent le chlorhydrate, le bromohydrate, l'hydrogénomaléate ou l'hydrogénooxalate.

Un composé particulièrement préféré est le composé identifié BF2649, à savoir le 3-(4-chlorophényl)propyl-3-pipéridinopropyl éther, également dénommé 1-{3-[3-(4-chlorophényl)propoxy]propyl}pipéridine.

Des antagonistes du récepteur H₃ ainsi que des agonistes inverses sont décrits dans les documents suivants EP 197 840, EP 494 010, WO93/14070 WO96/29315 US 6,248,765, WO92/15567, WO93/20061, WO93/20062, WO95/11894, US 5,486,526, WO93/12107, WO93/12108, WO95/14007, WO95/06037, WO97/29092, EP 680960, WO96/38141, WO96/38142, WO96/40126, Plazzi et al., Eur. J. Med. Chem. 1995, 30, 881, Clitherow et al., Bioorg. & Med. Chem. Lett. 6(7), 883-838 (1996), Wolin et al., Bioorg. & Med. Chem. Lett. ; 8, 2157 (1998), ainsi que WO 03/11856 A1 20030213 ; WO 03/24928 A2 20030327 ; WO 03/24929 A1 20030327 ; WO 02/79168 A1 20021010 ; WO 02/24695 A2 20020328 ; WO 02/12224 A2 20020214 ; WO 02/32893 A2 20020425 ; WO 02/12190 A2 20020214 ; US 2002183309 A1 20021205 ; WO 02/76925 A2 20021003 ; WO 02/12214 A2 20020214 ; WO 02/13821 A1 20020221 ; US 2002111340 A1 20020815 ; WO 02/06223 A1 20020124 : WO 01/81317 A1 20012201 ; WO 01/74810 A2 20011011 ; WO 01/74813 A2 20011011 ; WO 01/68652 A1 20010920 ; WO 01/68651 A1 20010920 ; WO 01/74815 A2 20011011 ; WO 01/74814 A1 20011011 ; WO 01/66534 A2 20010913 ; US 6166060 20001226 ; US 6100279 20000808 ;US 6034251 A 20000307 ; EP 978512 A1 20000209 ; WO 00/06254 A2 20000210 ;WO 00/42023 A1 20000720 ; WO 00/53596 A2 20000914 ; WO 00/23438 A1 20000427 ; WO 00/06552 A1 20000210 ; WO 00/64884 A1 20001102 ; WO 00/63208 A1 20001026 ; US 5932596 A 19990803 ; WO 99/05114 A2 19990204 ; US 6008240 A 19991228 ; WO 99/24421 A1 19990520 ; WO 99/42458 A1 19990826 ; WO 99/05141 A1 19990204 ; US 5990317 A 19991123; WO 99/05115 A1 19990204 ; US 5869479 A 19990209 ; US 5837718 A 19981117 ; US 5639775 A 19970617 ; WO 97/29092 A1 19970814 ; US 5463074 A- 19951031 ; WO 94/17058 A1 19940804 ; WO 93/12093 A1 19930624 ; US 5217986 A 19930608 ; WO 91/17146 A1 19911114.

Comme composés individuels, on peut citer :
1-(5-phenoxypentyl)-piperidine
1-(5-phenoxypentyl)-pyrrolidine
N-methyl-N-(5-phenoxypentyl)-ethylamine
1-(5-phenoxypentyl)-morpholine
N-(5-phenoxypentyl)-hexamethyleneimine
N-ethyl-N-(5-phenoxypentyl)-propylamine
1-(5-phenoxypentyl)-2-methyl-piperidine
1-(5-phenoxypentyl)-4-propyl-piperidine
1-(5-phenoxypentyl)-4-methyl-piperidine
1-(5-phenoxypentyl)-3-methyl-piperidine
1-acetyl-4-(5-phenoxypentyl)-piperazine
1-(5-phenoxypentyl)-3,5-trans-dimethyl-piperidine
1-(5-phenoxypentyl)-3,5-cis-dimethyl-piperidine
1-(5-phenoxypentyl)-2,6-cis-dimethyl-piperidine
4-carboethoxy-1-(5-phenoxypentyl)-piperidine
3-carboethoxy-1-(5-phenoxypentyl)-piperidine
1-[3-(4-cyclopropylcarbonylphenoxy) propyl]-piperidine
1-[3-(4-acetylphenoxy)-2-R-methylpropyl] piperidine
1-[3-(4-cyanophenoxy)propyl]-4-methylpiperidine
1-[3-(4-cyanophenoxy)propyl]-3-methylpiperidine
1-[3-(4-acetylphenoxy)-2-S-methylpropyl] piperidine
1-{3-[4-(3-oxobutyl)phenoxy] propyl}piperidine
1-[3-(4-cyano-3-fluorophenoxy)propyl] piperidine
1-[3-(4-nitrophenoxy)propyl]-3-methylpiperidine
1-[3-(4-cyanophenoxy)propyl]-2-methylpiperidine
1-[3-(4-nitrophenoxy)propyl]-2-methylpiperidine
1-[3-(4-nitrophenoxy)propyl]-4-methylpiperidine
1-[3-(4-cyanophenoxy)propyl]-2,6-dimethylpiperidine
1-[3-(4-propionylphenoxy)propyl]-3-methylpiperidine
1-[3-(4-cyclobutylcarbonylphenoxy)propyl] piperidine
1-[3-(4-cyclopentylcarbonylphenoxy) propyl]piperidine
1-[3-(4-cyanophenoxy)propyl]-cis-2-methyl-5-ethylpiperidine
1-[3-(4-cyanophenoxy)propyl]-trans-2-methyl-5-ethylpiperidine
1-[3-(4-cyanophenoxy)propyl]-cis-3,5-dimethylpiperidine
1-[3-(4-propionylphenoxy)propyl]-4-methylpiperidine
1-[3-(4-propionylphenoxy)propyl]-2-methylpiperidine
1-{3-[4-(1-hydroxypropyl)phenoxy]propyl}-3-methylpiperidine
1-{3-[4-(1-hydroxypropyl)phenoxy]propyl}-4-methylpiperidine
1-[3-(4-propionylphenoxy)propyl]-2-methylpiperidine
1-[3-(4-propionylphenoxy)propyl]-4-methylpiperidine methoxime
1-[3-(4-cyanophenoxy)propyl]-trans-3,5-dimethylpiperidine
1-[3-(4-cyclopropylcarbonylphenoxy) propyl] -trans-3,5-dimethyl piperidine
1-[3-(4-cyclopropylcarbonylphenoxy) propyl] -cis-3,5-dimethyl piperidine
1-[3-(4-carbomethoxyphenoxy)propyl] piperidine
1-[3-(4-propenylphenoxy)propyl]-2-methyl piperidine
1-[3-(4-propionylphenoxy)propyl]-2-methylpiperidine
1-{3-[4-(1-ethoxypropyl)phenoxy]propyl}-2-methyl piperidine
1-[3-(4-propionylphenoxy)propyl]-4-methylpiperidine
1-[3-(4-bromophenoxy)propyl]piperidine
1-[3-(4-nitrophenoxy)propyl]piperidine
1-[3-(4-N,N-dimethylsulfonamidophenoxy) propyl]piperidine
1-[3-(4-isopropylphenoxy)propyl]piperidine
1-[3-(4-sec-butylphenoxy)propyl]piperidine
1-[3-(4-propylphenoxy)propyl]piperidine
1-[3-(4-ethylphenoxy)propyl]piperidine
1-(5-phenoxypentyl)-1,2,3,6-tetrahydropyridine
1-[5-(4-nitrophenoxy)-pentyl]-pyrrolidine
1-[5-(4-chlorophenoxy)-pentyl]-pyrrolidine
1-[5-(4-methoxyphenoxy)-pentyl]-pyrrolidine
1-[5-(4-methylphenoxy)-pentyl]-pyrrolidine
1-[5-(4-cyanophenoxy)-pentyl]-pyrrolidine
1-[5-(2-naphthyloxy)-pentyl]-pyrrolidine
1-[5-(1-naphthyloxy)-pentyl]-pyrrolidine -
1-[5-(3-chlorophenoxy)-pentyl]-pyrrolidine
1-[5-(4-phenylphenoxy)-pentyl]-pyrrolidine
1-{5-[2-(5,6,7,8-tetrahydronaphthyl)-oxy]-pentyl}-pyrrolidine
1-[5-(3-phenylphenoxy)-pentyl]-pyrrolidine
1-(5-phenoxypentyl)-2,5-dihydropyrrole
1-{5-[1-(5,6,7,8-tetrahydronaphthyl)-oxy]-pentyl}-pyrrolidine
1-(4-phenoxybutyl)-pyrrolidine
1-(6-phenoxyhexyl)-pyrrolidine
1-(5-phenylthiopentyl)-pyrrolidine
1-(4-phénylthiobutyl)-pyrrolidine
1-(3-phenoxypropyl)-pyrrolidine
1-[5-(3-nitrophenoxy)-pentyl]-pyrrolidine
1-[5-(4-fluorophenoxy)-pentyl]-pyrrolidine
1-[5-(4-n itrophenoxy)-pentyl]-3-methyl-piperid ine
1-[5-(4-acetylphenoxy)-pentyl]-pyrrolidine
1-[5-(4-aminophenoxy)-pentyl]-pyrrolidine
1-[5-(3-cyanophenoxy)-pentyl]-pyrrolidine
N-[3-(4-nitrophenoxy)-propyl]-diethylamine
N-[3-(4-cyanophenoxy)-propyl]-diethylamine
1-[5-(4-benzoylphenoxy)-pentyl]-pyrrolidine
1-{5-[4-(phenylacetyl)-phenoxy]-pentyl}-pyrrolidine
N-[3-(4-acetylphenoxy)-propyl]-diethylamine
1-[5-(4-acetamidophenoxy)-pentyl]-pyrrolidine
1-[5-(4-phenoxyphenoxy)-pentyl]-pyrrolidine
1-[5-(4-N-benzamidophenoxy)-pentyl]-pyrrolidine
1-{5-[4-(1-hydroxyethyl)-phenoxy]-pentyl}-pyrrolidine
1-[5-(4-cyanophenoxy)-pentyl]-diethylamine
1-[5-(4-cyanophenoxy)-pentyl]-piperidine
N-[5-(4-cyanophenoxy)-pentyl]-dimethylamine
N-[2-(4-cyanophenoxy)-ethyl]-diethylamine
N-[3-(4-cyanophenoxy)-propyl]-dimethylamine
N-[4-(4-cyanophenoxy)-butyl]-diethylamine
N-[5-(4-cyanophenoxy)-pentyl]-dipropylamine
1-[3-(4-cyanophenoxy)-propyl]-pyrrolidine
1-[3-(4-cyanophenoxy)-propyl]-piperidine
N-[3-(4-cyanophenoxy)-propyl]-hexamethyleneimine
N-[6-(4-cyanophenoxy)-hexyl]-diethylamine
N-[3-(4-cyanophenoxy)-propyl]-dipropylamine
N-3-[4-(1-hydroxyethyl)-phenoxy]-propyl-diethylamine
4-(3-diethylaminopropoxy)-acetophenone-oxime
1-[3-(4-acetylphenoxy)-propyl]-piperidine
1-[3-(4-acetylphenoxy)-propyl]-3-methyl-piperidine
1-[3-(4-acetylphenoxy)-propyl]-3,5-trans-dimethyl-piperidine
1-[3-(4-acetylphenoxy)-propyl]-4-methyl-piperidine
1-[3-(4-propionylphenoxy)-propyl]-piperidine
1-[3-(4-acetylphenoxy)-propyl]-3,5-cis-dimethyl-piperidine
1-[3-(4-formylphenoxy)-propyl]-piperidine
1-[3-(4-isobutyrylphenoxy)-propyl]-piperidine
N-[3-(4-propionylphenoxy)-propyl]-diethylamine
1-[3-(4-butyrylphenoxy)-propyl]-piperidine
1-[3-(4-acetylphenoxy)-propyl]-1,2,3,6-tetrahydropyridine
α-(4-Acetylphenoxy)-α'-(4-methylpiperidino)p-xylol
α-(4-Acetylphenoxy)-α'-(3,5-cis-dimethylpiperidino)p-xylol
α-(4-Acetylphenoxy)-α'-(3,5-*trans*-dimethylpiperidino)p-xylol
α-(4-Acetylphenoxy)-α'-(2-methylpyrrolidino)p-xylol
α-(4-Cyclopropylcarbonylphenoxy)-α'-piperidino-p-xylol
α-(4-Cyclopropylcarbonylphenoxy)-α'-(4-méthylpiperidino)p-xylol
α-(4-Cyclopropylcarbonylphenoxy)-α'-pyrrolidino-p-xylol
3-Phenylpropyl 3-(4-methylpiperidino)propyl ether
3-Phenylpropyl 3-(3,5-*cis*-dimethylpiperidino)propyl ether
3-Phenylpropyl 3-(3,5-*trans*-dimethylpiperidino)propyl ether
3-Phenylpropyl 3-(3-methylpiperidino)propyl ether
3-Phenylpropyl 3-pyrrolidinopropyl ether
3-(4-Chlorophenyl)propyl 3-(4-methylpiperidino)propyl ether
3-(4-Chiorophenyl)propyl 3-(3,5-*cis*-dimethylpiperidino)propyl ether
3-(4-Chlorophenyl)propyl3-(3,5-*trans*-dimethylpiperidino)propyl ether
4-(6-Piperidinohexylamino)quinoline
2-Methyl 4-(3-piperidinopropylamino)quinoline
2-Methyl 4-(6-piperidinohexylamino)quinoline
7-Chloro-4-(3-piperidinopropylamino)quinoline
7-Chloro-4-(4-piperidinobutylamino)quinoline
7-Chloro-4-(8-piperidinooctylamino)quinoline
7-Chloro-4-(10-piperidinodecylamino)quinoline
7-Chloro-4-(12-piperidinododecylamino)quinoline
7-Chloro-4-(4-(3-piperidinopropoxy)phenylamino)quinoline
7-Chloro-4-(2-(4-(3-piperidinopropoxy)phenyl)ethylamino)quinoline
4-(6-Piperidinohexanoyl)phenyl 3-piperidinopropyl ether
5-Nitro-2-(5-piperidinopentylamino)pyridine
3-Nitro-2-(6-piperidinopentylamino)pyridine
5-Amino-2-(6-piperidinopentylamino)pyridine
2-(6-Piperidinohexylamino)quinoline
*N*-(4-Chlorobenzyl)-*N'*-cyclohexyl-3-piperidinopropyl isothiourea
2-(6-Piperidinohexylamino)benzothiazole
10-Piperidinodecylamine
3-Phenylpropyl 3-(*N,N*-diethylamino)propyl ether
N-(3-(N,N-Diethylamino)propyl)N'-phenylurea
N-Cyclohexylmethyl-N'-(3-piperidinopropyl)guanidine
N-(4-Bromobenzyl)-N'-(4-piperidinobutyl)sulphamide
3-Chloro-N-(4-piperidinobutyl)-N-methyl-benzene sulphonamide
N-(4-Chlorobenzyl)-2-(4-piperidinomethyl) phenyl) ethan amidine
1-(5-Cyclohexylpentanoyl)-1,4-bipiperidine
cis-1-(6-Cyclohexyl-3-hexen-1-yl)piperidine
trans-1-(6-Cyclohexyl-3-hexen-1-yl)piperidine
1-(2-(5,5-Dimethyl-1-hexin-1-yl)cyclopropyl)piperidine
3,3-Dimethylbutyl 3-piperidinopropyl ether
3-Phenylpropyl 3-piperidinopropyl ether
3-(4-Chlorophenyl)propyl 3-piperidinopropyl ether
2-Benzothiazolyl 3-piperidinopropyl ether
3-Phenylpropyl 3-(4-methylpiperidino)propyl ether
3-Phenylpropyl 3-(3,5-cis-dimethylpiperidino)propyl ether
3-Phenylpropyl 3-(3,5-trans-dimethylpiperidino)propyl ether
3-Phenylpropyl 3-(3-methylpiperidino)propyl ether
3-Phenylpropyl 3-pyrrolidinopropyl ether
3-(4-Chlorophenyl)propyl 3-(4-methylpiperidino)propyl ether
3-(4-Chloro phenyl) propyl 3-(3,5-cis-dimethyl piperidino) propyl ether
3-(4-Chloro phenyl) propyl 3-(3,5-trans-dimethyl piperidino) propyl ether
3-Phenylpropyl 3-(N,N-diethylamino)propyl ether
N-Phenyl-3-piperidinopropyl carbamate
N-Pentyl-3-piperidinopropyl carbamate
(S)-(+)-N-[2-(3,3-Dimethyl)butyl]-3-piperidinopropyl carbamate
3-Cyclopentyl-N-(3-(1-pyrrolidinyl)propyl)propanamide
N-Cyclohexyl-N'-(1-pyrrolidinyl-3-propyl)urea
2-((2-Piperidinoethyl)amino)benzothiazole
5-Piperidinopentylamine
2-Nitro-5-(6-piperidinohexyl)pyridine
3-Nitro-2-(6-piperidinohexylamino)pyridine
2-(6-Piperidinohexylamino)pyrimidine
N-(6-Phenylhexyl)piperidine
N-(3-(N,N-Diethylamino)propyl)N'-phenylurea
N-Cyclohexylmethyl-N'-(3-piperidinopropyl)guanidine
3-(3,4-Dimethoxyphenyl)propyl 3-piperidinopropyl ether
3-(4-Fluorophenyl)propyl 3-pyrrolidinopropyl ether
3-(4-Hydroxy-3-methoxyphenyl)propyl 3-piperidinopropyl ether
3-(3-Hydroxy-4-methoxyphenyl)propyl 3-piperidinopropyl ether
3-(4-Fluoro-3-methoxyphenyl)propyl 3-piperidinopropyl ether
3-(4-Fluoro-3-methoxyphenyl)propyl 3-pyrrolidinopropyl ether
1-[3-(4-butylphenoxy)propyl]piperidine
1-[3-(4-phenylphenoxy)propyl]piperidine
1-{3-[4-(3-oxobutyl)phenoxy]propyl}-3, 5-trans-dimethyl-piperidine
1-{3-[4-(3-oxobutyl)phenoxy]propyl}-3,5-cis-dimethyl-piperidine
1-[3-(4-butylphenoxy)propyl]-3,5-trans-dimethyl-piperidine
1-[3-(4-butylphenoxy)propyl]-3,5-cis-dîmethyl-piperidine
1-[3-(4-phenylphenoxy)propyl]-3,5-trans-dimethyl-piperidine
1-[3-(4-phenylphenoxy)propyl]-3,5-cis-dimethyl-piperidine
ou ses sels pharmaceutiquement acceptables, hydrates, ou sels hydratés, ou les structures crystallines polymorphiques de ces composés ou leurs isomères optiques, racémates, diastéréoisomères ou énantiomères.

Comme composés individuels, on peut notamment citer :
3-phénylpropyl 3-pipéridinopropyl éther ;
1-[5-(4-acétamidophénoxy)-pentyl]-pyrrolidine ;
1-(3-[4-oxobutyl)phénoxyl]propyl)pipéridine ;
1-(3-[4-(1-hydroxypropyl)phénoxy]propyl)-3-méthylpipéridine ;
1-3-[4-(1-hydroxypropyl)phénoxy]propyl)-4-méthylpipéridine ;
1-[3-(4-cyanophénoxy)-propyl]-pipéridine ;
N-[3-(4-cyanophénoxy)-propyl]-hexaméthylèneimine ;
1-[3-(4-acétylphénoxy-propyl]-3-méthyl-pipéridine ;
1-(3-[4-(1-éthoxypropyl)phénoxy]propyl)-2-méthylpipéridine oxime ;
1-[3-(4-bromophénoxy)propyl]pipéridine ;
1-[3-(4-isopropylphénoxy)propyl]pipéridine ;
1-[3-(4-sec-butylphénoxy)propyl]pipéridine ;
1-[3-(4-propylphénoxy)propyl]pipéridine ;
1-[3-(4-éthylphénoxy)propyl]pipéridine .

Ces composés sont décrits dans la demande PCT/EP99/05744 (WO00/06254). Ils peuvent être préparés par application ou adaptation des méthodes décrites dans cette demande, ou de toute autre méthode à la portée de l'homme de l'art.

Notamment, les produits suivants ont été préparés selon ces méthodes et présentent les caractéristiques suivantes :
3-(3,4-Dimethoxyphenyl)propyl 3-piperidinopropyl ether (sel d'oxalate): F = 111°C
3-(4-Fluorophenyl)propyl 3-pyrrolidinopropyl ether (sel d'oxalate): F = 106°C
3-(4-Hydroxy-3-methoxyphenyl)propyl 3-piperidinopropyl ether (sel d'oxalate): F = 110-111°C
3-(3-Hydroxy-4-methoxyphenyl)propyl 3-piperidinopropyl ether (sel d'oxalate): F = 101-103°C
3-(4-Fluoro-3-methoxyphenyl)propyl 3-piperidinopropyl ether (sel d'oxalate) : F = 125-126°C
3-(4-Fluoro-3-methoxyphenyl)propyl 3-pyrrolidinopropyl ether (sel d'oxalate): F = 87-88°C
1-{3-[4-(3-oxobutyl)phenoxy]propyl}-3,5-cis-dimethyl-piperidine (sel d'oxalate): F = 114°C
1-{3-[4-(3-oxobutyl)phenoxy]propyl}-3,5-trans-dimethyl-piperidine (sel d'oxalate): F = 120°C

Les proportions du composé (A) par rapport au composé (B) sont de préférences de 5 à 100 mg, plus préférentiellement de 5 à 80 mg de composé (B) pour 0,5 à 50 mg de composé (A).

Pour le composé (A), on préférera utiliser, au niveau de la posologie, des dosages identiques ou similaires aux dosages habituellement utilisés pour les traitements antipsychotiques ou antidépresseurs.

Cependant, dans une forme de réalisation particulière, et compte tenu d'un effet anti-psychotique ou antidépresseur propre au composé (B), la dose de composé (A) peut être abaissée, par exemple à 50-90%, de la dose usuelle.

Pour le composé (B), la dose utilisée peut correspondre à une dose habituelle telle qu'enseigné dans la demande de brevet précitée ou de 5-80 mg, par exemple 20-50 mg du composé BF 2649.

Cependant, dans une forme de réalisation perfectionnée, cette dose peut être abaissée, en étant de préférence égale au moins à 10 à 15% de la dose habituelle, par exemple 50% de la dose habituelle.

Le médicament selon l'invention peut être administré par toute voie d'administration convenant à l'administration antipsychotique ou à titre d'antidépresseur. De préférence, une administration orale est prévue.

En conséquence le médicament peut se composer sous forme de comprimés, de gélules, de poudre ou de toute forme pour préparation orale solide ou sous toute forme de préparation buvable. La formulation peut être associée à un véhicule pharmaceutiquement acceptable, par exemple pour la préparation de comprimés ou de gélules ou pour une préparation buvable telle que les véhicules utilisés de façon tout à fait classique dans la pharmacopée et notamment déjà utilisés déjà pour les neuroleptiques décrits dans l'invention.

Pour déterminer les neuroleptiques ou autres médicaments antipsychotiques et/ou antidépresseurs (A) susceptibles d'être utilisés dans les médicaments selon l'invention, on peut rechercher par les tests *in vitro,* éventuellement *in vivo,* les agents psychotropes bloquant les récepteurs H₁- De tels tests sont décrits par exemple dans Quach et al., Eur. J. Pharmacol., 1979, 60, 391.

Ainsi, parmi ces médicaments psychotropes, on peut décrire, outre les antipsychotiques et antidépresseurs décrits ci-dessus, la carbamazépine.

L'invention a également pour objet l'utilisation d'un antagoniste et/ou agoniste inverse du récepteur H₃ de l'histamine, c'est-à-dire d'un composé (B) tel que décrit ci-dessus, pour la préparation d'un médicament destiné à être administré en complément d'un traitement psychiatrique par un antipsychotique ou un antidépresseur (A) tel que décrit ci-dessus pour prévenir ou corriger les effets indésirables d'un tel traitement sur la vigilance occasionnés ou susceptibles d'être occasionnés par ledit traitement.

Selon un autre aspect préféré, ledit effet indésirable est en particulier l'épilepsie et/ou les convulsions. L'épilepsie et/ou les convulsions ont notamment été observées à titre d'effet induit de l'olanzapine, ou de la rispéridone, clozapine, mirtazapine. Selon un autre aspect préféré, la présente invention concerne donc l'utilisation d'un antagoniste et/ou agoniste inverse du récepteur H₃ de l'histamine, c'est-à-dire d'un composé (B) tel que décrit ci-dessus, pour la préparation d'un médicament destiné à être administré en complément d'un traitement psychiatrique par un antipsychotique ou un antidépresseur (A) pour prévenir ou corriger l'épilepsie et/ou les convulsions.

L'invention a également pour objet l'utilisation d'un tel composé pour la préparation d'un médicament destiné à être administré en complément d'un traitement par antipsychotique ou antidépresseur (A) afin de potentialiser les effets thérapeutiques dudit traitement sur la sphère cognitive.

L'invention a également pour objet un antagoniste et/ou agoniste inverse du récepteur H₃ de l'histamine (B) tel que défini plus haut pour prévenir ou corriger les effets indésirables d'un traitement psychiatrique par un antipsychotique ou un antidépresseur (A) présentant un effet antagoniste du récepteur de l'histamine H₁ et choisi parmi l'olanzapine, la clozapine, la rispéridone, la quiétapine, la mirtazapine, l'aripiprazole, la paroxétine et l'amitryptyline, sur la prise de poids occasionnée ou susceptible d'être occasionnée par ledit traitement pour administration en complément d'un tel traitement.

L'invention concerne également l'utilisation d'un composé (A) et d'un composé (B) tels que définis ci-avant pour la préparation d'un médicament pour prévenir et/ou traiter une pathologie choisie parmi : la schizophrénie, la dépression, la psychose, les désordres mentaux, la manie, les troubles affectifs bipolaires.

Les utilisations selon l'invention peuvent comporter la fabrication d'un médicament comprenant, à la fois, le composé (A) et le composé (B) tels que définis ci-dessus.

Ces utilisations peuvent également prévoir la fabrication d'un ensemble de compositions distinctes, l'une contenant le composé (A) et l'autre le composé (B), en association dans une même présentation.

Enfin, les utilisations selon l'invention peuvent comporter la fabrication d'un médicament destiné à prévenir ou corriger lesdits effets indésirables dans le traitement précité, ou potentialiser l'effet thérapeutique sur la sphère cognitive dans un tel traitement, sous forme d'un médicament comportant uniquement ou essentiellement le composé (B) comme principe actif.

Un tel médicament est fabriqué de préférence sous une forme à administration orale, analogue à celles décrites ci-dessus.

Le dosage d'un tel médicament comporte, de préférence, une dose de composé (B) égale ou inférieure à la dose normalement préconisée pour l'utilisation déjà connue du composé (B). De préférence, cette dose est comprise entre 15 ou 20% et jusqu'à 100% de cette dose. Cependant on peut également prévoir des doses supérieures à cette dose normalement préconisée pour le composé (B).

Le composé (B) peut donc être administré soit sous forme d'une association avec le composé antipsychotique ou antidépresseur (A), formant un médicament unique, ou bien encore, être administré séparément, soit concomitamment, soit indépendamment, par exemple, avec un décalage dans le temps.

De façon préférée, le rythme d'administration du composé (B) est identique ou similaire au rythme d'administration du composé (A).

D'autres avantages et caractéristiques de l'invention apparaîtront à la lecture des exemples suivants non limitatifs.

### Exemple 1 : Association olanzapine - composé BF2649

On prépare une formulation orale sous forme de comprimés ou gélules ou préparation buvable comprenant de 5 à 80 mg de composé BF2649 et 3 à 20 mg d'olanzapine.

Une telle formulation est administrée de préférence une fois par jour.

De préférence, la formulation associe 20 à 60 mg de composé BF2649 à 20 mg d'olanzapine pour un adulte.

### Exemple 2 : Association rispéridone - composé BF2649

On prépare une formulation orale sous forme de comprimés ou gélules ou préparation buvable comprenant de 5 à 80 mg de composé BF2649 et 0,5 à 10 mg de rispéridone.

Une telle formulation est administrée de préférence une fois par jour.

De préférence, la formulation associe 20 à 60 mg de composé BF2649 à 10 mg de rispéridone pour un adulte.

### Exemple 3 : Association aripiprazole - composé BF2649

On prépare une formulation orale sous forme de comprimés ou gélules ou préparation buvable comprenant de 5 à 80 mg de composé BF2649 et 10 à 30 mg d'aripiprazole.

Une telle formulation est administrée de préférence une fois par jour.

De préférence, la formulation associe 20 à 60 mg de composé BF2649 à 10 à 30 mg d'aripiprazole pour un adulte.

Les associations du composé BF2649 et de clozapine ou de quiétapine ou d'un antidépresseur comme la mirtazapine sont réalisés de façon similaire.

### Exemple 4 : Utilisation du composé BF2649

Pour préparer un médicament destiné à être utilisé pour prévenir ou corriger les effets indésirables d'un traitement antipsychotique ou antidépresseur, on fabrique une composition à administration orale, notamment sous forme de comprimés gélules ou préparations buvables, comprenant une dose de 20 à 60 mg de composé BF2649.

L'invention permet ainsi de prévenir ou de corriger la prise de poids par des antipsychotiques, antidépresseurs et autres agents psychotropes, ainsi que ces autres effets indésirables en administrant simultanément un antagoniste/agoniste inverse du récepteur H₃ de l'histamine.

Sans vouloir être liés par la théorie, les présents inventeurs pensent pouvoir expliquer cet effet inattendu par les considérations suivantes :
1) les antagonistes/agonistes inverses H₃ augmentent considérablement la libération d'histamine cérébrale et, à ce titre, protègent le récepteur H₁ du blocage par de nombreux agents psychotropes par un processus de compétition,
2) les antagonistes/agonistes inverses H₃ tendent, par eux-mêmes, à diminuer la prise alimentaire (Sakata et al. Nutrition, 1997, 5, 403).
3) L'action des antagonistes/agonistes inverses H₃ ne s'oppose en aucune manière à l'action des antipsychotiques ou antidépresseurs et serait même de nature à la favoriser. En effet, par eux-mêmes, i) ils s'avèrent actifs sur certains « modèles » animaux de schizophrénie (blocage de l'activation psychomotrice induite par l'amphétamine sur les antagonistes NMDA), ii) ils exercent un effet pro-cognitif recherché dans le traitement aussi bien de la schizophrénie que de la dépression.
4) Leur effet éveillant qui est mis en évidence chez l'homme, est de nature à s'opposer à l'effet sédatif indésirable de nombreux agents psychotropes. Il a aussi été montré qu'un antagoniste/agoniste inverse H₃ administré chez l'homme induit une augmentation des ondes alpha de haute fréquence, connues pour être associées à des processus cognitifs, indiquant par là que ce médicament est utile pour augmenter l'effet pro-cognitif des antipsychotiques, recherché dans les psychoses et désordres mentaux associés.

Ces observations ont été confirmées par les résultats expérimentaux suivants :

### Exemple 5 : Activité des combinaisons selon l'invention

### Données animales :

Le composé BF2649 a été administré à la dose de 5mg/kg par voie intrapéritonéale, seul ou en combinaison avec 0.15 mg/kg d'olanzapine per os chez des souris pour lesquelles une hyperlocomotion a été induite (par administration de 0.2mg/kg de dizocilpine).

Les données expérimentales montrent que l'administration separée du BF2649 ou de l'olanzapine n'a que des effets marginaux sur l'hyperlocomotion induite par dizocilpine alors que l'administration des 2 composés en association prévient totalement l'effet hyperlocomoteur de 0.2mg/kg de dizocilpine.

Ces résultats établissent que l'antagoniste H3 potentialise les effets de l'olanzapine sur un certain nombre de tests d'antipsychotiques.

### Données humaines :

Un groupe de 6 mâles volontaires a reçu par voie orale,successivement et à une semaine d'intervalle :1) un placebo 2) de l'olanzapine à la dose de 5mg 3) du BF 2649 à la dose de 60 mg 4)une combinaison d'olanzapine et de BF 2649 aux doses indiquées ci-dessus.

Les sujets ont alors été analysés pendant une période de 24h en ce qui concerne des paramètres de vigilance (EEG quantitatif), de cognition (tests psychométriques variés) et de satiété (questionnaires d'autoévaluation des sensations de faim, désir de manger, plénitude gastrique). En outre, on a procédé à l'analyse répétée des taux des deux médicaments dans le sang au cours du nycthemère.

On a constaté que l'olanzapine, administrée seule, induisait une profonde sédation accompagnée d'une diminution des ondes rapides de l'EEG au profit des ondes lentes, une profonde altération des tests psychométriques notamment d'attention, et une augmentation très marquée des sensations de faim. Sur ces différentes manifestations, à la base des effets secondaires de l'antipsychotique, on a constaté des effets inverses avec le BF 2649 et, surtout une prévention parfois totale des effets de l'olanzapine lorsqu'il était associé à cette dernière. Cette prévention dans le domaine de la satiété a été confirmée par une normalisation de la sécrétion de leptine altérée chez ces sujets par l'administration d'olanzapine seule.

En outre, l'association a été parfaitement tolérée et n'a pas conduit à des modifications marquées des taux plasmatiques de chacun des médicaments.

L'effet particulièrement spectaculaire dans le domaine de l'appétit indique que l'association est de nature à prévenir le développement de la prise de poids, du diabète de type Il et du syndrome métabolique qui accompagnent souvent le traitement chronique par les antipsychotiques du type olanzapine.

Les effets sur l'EEG quantitatif indiquent que les effets procognitifs des antagonistes H3 sont maintenus chez un sujet recevant un médicament antipsychotique du type olanzapine et que les effets proconvulsivants de ces derniers seront prévenus par l'association avec un antagoniste H3.

## Revendications

1. Nouveau médicament comprenant, dans un véhicule pharmaceutiquement acceptable, un anti-psychotique ou un antidépresseur (A), présentant seul un effet indésirable de prise de poids corporel ou de sédation et un antagoniste et/ou agoniste inverse (B) du récepteur H₃ de l'histamine, ledit antipsychotique ou antidépresseur étant présent dans le médicament en une quantité thérapeutiquement efficace pour l'effet antipsychotique ou antidépresseur recherché, et ledit antagoniste et/ou agoniste inverse du récepteur H₃ de l'histamine étant présent en une quantité thérapeutiquement efficace pour assurer l'un au moins des trois effets suivants : suppression ou au moins limitation de l'effet indésirable de prise de poids, suppression ou limitation de l'effet indésirable sur la vigilance, augmentation de l'effet pro-cognitif du traitement, tel que :
- l'agent (A) est choisi parmi les agents présentant un effet antagoniste du récepteur de l'histamine H₁ **choisis parmi l'olanzapine, la clozapine, la rispéridone, la quiétapine, la mirtazapine, l'aripiprazole** ; et
- l'agent (B) répond à la formule (II) 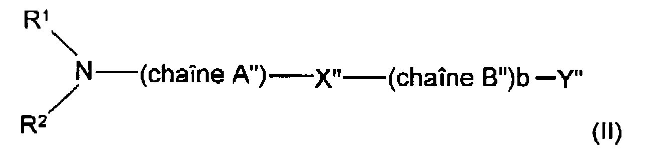 dans laquelle :
b=0 ou 1
i) R¹ et R², identiques ou différents, représentent chacun, indépendamment,
- un alkyle en C1-C6 ou un cycloalkyle,
ou pris ensemble avec l'atome d'azote auquel ils sont fixés,
- un cycle azoté saturé 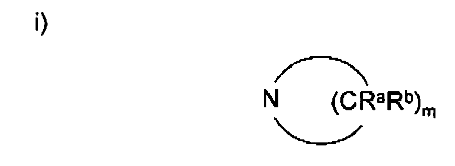 avec m de 2 à 8 ou
un cycle azoté insaturé non aromatique 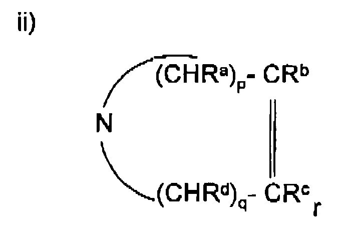
avec p et q indépendamment de 0 à 3 et r de 0 à 4, sous la condition que p et q ne soient pas simultanément 0 et que 2 ≤ p+q+r ≤8,
R^{a-d} étant indépendamment un atome d'hydrogène ou un groupe alkyle en C1-C6, cycloalkyle ou carboalkoxy ou
- un groupe morpholino ou
- un groupe piperazino N-substitué 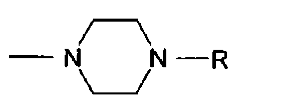 R étant un groupe alkyle en C1-C6, cycloalkyle, carboalkoxy, aryle, arylalkyle, alcanoyle ou un groupe aroyle,
ii) la chaîne A" est choisie dans un groupe alkylène linéaire, ramifié saturé ou insaturé -(CH₂)_{n"}- dans lequel n" est un entier de 1 à 8 ; un groupe alkénylène linéaire ou ramifié comprenant de 1 à 8 atomes de carbone ; et un groupe alkynylène linéaire ou ramifié comprenant de 1 à 4 atomes de carbone ;
iii) le groupe X" est choisi parmi -OCONH-, OCON(alkyle)-, -OCON(alcène)-, -OCO-, -OCOSNH-, -CH₂-, -O-, -OCH₂CO-, -S-, -CO-, -CS-, une amine ou un alkyle saturé ou insaturé ;
iv) la chaîne B" est choisie parmi les alkylènes, linéaires, ramifiés, saturés ou insaturés comprenant de 1 à 8 atomes de carbone ; et -(CH₂)_{n"}(hétéroatome)- où l'hétéroatome est, de préférence, un atome d'oxygène ou de soufre ; n" étant un entier de 1 à 5 ; et
v) le groupe Y" représente un groupe phényle non substitué ou mono ou polysubstitué par un ou plusieurs substituants identiques ou différents choisis parmi les atomes d'halogène, OCF₃, CHO, CF₃, SO₂N(alkyle)₂ tel que SO₂N(CH₃)₂, NO₂, S(aryle), SCH₂(phényle), un alcène linéaire ou ramifié, un alcyne linéaire ou ramifié éventuellement substitué par un radical trialkyle silyle, -O(alkyle), -O(aryle), -CH₂CN, une cétone, un aldéhyde, une sulfone, un acétal, un alcool, un alkyle en C₁-C₆, -CH=CH-CHO, -C(alkyle)=N-OH, -C(alkyle)=N-O(alkyle) et d'autres dérivés cétoniques, -CH=NOH, -CH=NO(alkyle) et d'autres dérivés aldéhydes, -C(alkyle)=NH-CONH₂, et O-phényle ou le groupe -OCH₂(phényle), -C(cycloalkyle)-NOH, -C(cycloalkyl)=N-O(alkyle) ; un hétérocycle éventuellement substitué, un cycloalkyle ; un groupe bicyclique et de préférence un groupe norbomyle ; un noyau phényle fusionné à un hétérocycle comprenant un hétéroatome d'azote ou à un carbocycle ou à un hétérocycle présentant une fonction cétonique ; un alkyle en C₁-C₆ linéaire ou ramifié; un alcyne linéaire ou ramifié comprenant de 1 à 8 atomes de carbone et notamment 1 à 5 atomes de carbone ; un alkyle linéaire ou ramifié mono ou polysubstitué par des groupes phényles qui sont non substitués ou mono ou polysubstitués ; une phénylalkyle cétone dans laquelle le groupe alkyle est linéaire ou ramifié ou cyclique ; une benzophénone substituée ou non substituée ; un phényle alcool substitué ou non substitué, linéaire, ramifié ou cyclique ; un alcène linéaire ou ramifié ; un groupe pipéridyle ; un groupe phényle cycloalkyle ; un groupe polycyclique, notamment un groupe fluorényle, un groupe naphtyle ou polyhydronaphtyle ou un groupe indanyle ; un groupe phénol ; une cétone ou un dérivé cétonique ; un groupe diphényle, un groupe phénoxyphényle ; un groupe benzyloxyphényle, -CN, -alkyle, -aryle, -alkyleCOAlkyle, -COOAlkyle, -COalkyle, -COaryle, -COaralkyle, -COcycloalkyle, -OH, -alkyle(OH), -alkyle(Oalkyle), -NHCOAlkyle, -NH₂,
ou ses sels pharmaceutiquement acceptables, hydrates, ou sels hydratés, ou les structures crystallines polymorphiques de ces composés ou leurs isomères optiques, racémates, diastéréoisomères ou énantiomères.

2. Médicament selon la revendication 1 dans lequel ledit antipsychotique ou antidépresseur (A) est l'olanzapine.

3. Médicament selon la revendication 1 ou 2 tel que (B) est de formule (IIa) 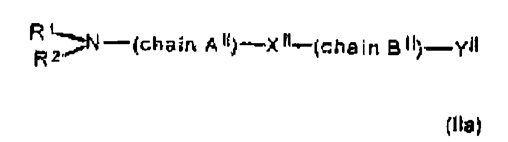 dans laquelle:
R¹ et R² , identiques ou différents, représente chacun indépendamment
• un cycle azoté saturé
i) 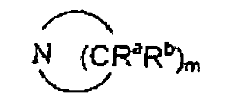 avec m compris entre 2 to 8,
R^{a-b} étant choisis indépendamment parmi un atome d'hydrogène ou un radical alkyle en C1-C4, et
ii) la chaîne A" est choisie parmi un groupe alkylelinéaire -(CH₂)_{nII}- pour lequel n_{II} est 3 ;
iii) le groupe X" est -O- ;
iv) la chaîne B^{II} est un groupe alkyle linéaire comprenant 3 atomes de carbone; et
v) le group Y^{II} représente un groupe phényle, non-substitué ou mono- ou polysubstitué avec un ou plusieurs substituants identiques ou différents choisis parmi les atomes d'halogène, OCF₃, CHO, CF₃, SO₂N(alkyl)₂ tel que SO₂N(CH₃)₂, NO₂, S(aryl), SCH₂(phenyl), -alkyleCOAlkyle, -COOAlkyle, -COalkyle, -COaryle, -COaralkyle, -COcycloalkyle, -alkyle(OH), -alkyle(Oalkyle), -Oaryle, -NHCOAlkyle, -Oalkyle, -C(alkyle)=N-OH , -C(alkyle)=N-O(alkyle), -NO2, -NH2, un alkène linéaire ou ramifié, un alkyne linéaire ou ramifié éventuellement substitué avec un radical trialkylsilyle, -O(alkyl), -O(aryl), -CH₂CN, une cétone, un aldéhyde, une sulphone, un acétal, un alcool, un alkyl en C1-C4, -CH=CH-CHO, -C(alkyl)=N-OH, -C(alkyl)=N-O(alkyl) et autres dérivés cétoniques, -CH=NOH, -CH=NO(alkyl), et autres dérivés aldéhydes, -C(alkyl)=NH-NH-CONH₂, O-phényl or -OCH₂(phényl), -C(cycloalkyl)=NOH, -C(cycloalkyl)=N-O(alkyl) ;
ou ses sels pharmaceutiquement acceptables, hydrates, ou sels hydratés, ou les structures crystallines polymorphiques de ces composés ou leurs isomères optiques, racémates, diastéréoisomères ou énantiomères.

4. Médicament **selon l'une quelconque des revendications** précédentes, dans lequel le groupement Y" est un groupe phényle substitué par un atome d'halogène.

5. Médicament **selon l'une quelconque des revendications précédentes** dans lequel ledit composé (B) est le 3-(4-chlorophényl)propyl-3-pipéridinopropyl éther (BF2649), ou ses sels pharmaceutiquement acceptables, hydrates, ou sels hydratés, ou les structures crystallines polymorphiques de ces composés ou leurs isomères optiques, racémates, diastéréoisomères ou énantiomères.

6. Médicament **selon l'une quelconque des revendications précédentes** dans lequel les proportions du composé (A) par rapport au composé (B) sont de 5 à 100 mg de composé (B) pour 0,5 à 50 mg de composé (A).

7. Médicament **selon l'une quelconque des revendications précédentes** adapté à une administration orale.

8. Médicament selon la revendication 7 sous forme de comprimés, de gélules, de poudre ou de préparation buvable.

9. Médicament **selon l'une quelconque des revendications précédentes,** notamment sous forme de comprimé, gélule ou préparation buvable associant 5 à 80 mg de composé (BF2649), à 3 à 20 mg d'olanzapine.

10. Médicament **selon l'une quelconque des revendications précédentes,** notamment sous forme de comprimé, gélule ou préparation buvable associant 5 à 80 mg de composé (BF2649), à 0,5 à 10 mg de rispéridone.

11. Médicament **selon l'une quelconque des revendications précédentes,** notamment sous forme de comprimé, gélule ou préparation buvable associant 5 à 80 mg de composé (BF2649), à 10 à 30 mg d'aripiprazole.

12. Antagoniste et/ou agoniste inverse du récepteur H₃ de l'histamine (B) tel que défini selon l'une quelconque des revendications précédentes pour utilisation dans la prévention ou la connection des effets indésirables d'un traitement psychiatrique par un antipsychotique ou un antidépresseur (A) selon l'une quelconque des revendications précédentes sur la vigilance occasionnés ou susceptibles d'être occasionnés par ledit traitement ou pour potentialiser les effets thérapeutiques dudit traitement sur la sphère cognitive, pour administration en complément d'un tel traitement.

13. Antagoniste et/ou agoniste inverse du récepteur H₃ de l'histamine (B), tel que défini selon l'une quelconque de revendications 1 à 11 pour utilisation dans la prévention ou la correction de l'épilepsie et/ou des convulsions occasionnés ou susceptibles d'être occasionnés par un traitement psychiatrique par un antipsychotique ou un antidépresseur (A) tel que défini selon l'une quelconque des revendications 1 à 11 pour administration en complément d'un tel traitement.

14. Antagoniste et/ou agoniste inverse du récepteur H₃ de l'histamine (B), tel que défini selon l'une quelconque de revendications 1 à 11 pour utilisation dans la potentialisation de l'effet thérapeutique sur la sphère cognitive d'un traitement psychiatrique par un antipsychotique ou un antidépresseur (A) tel que défini selon l'une quelconque des revendications 1 à 11, pour administration en complément d'un tel traitement.

15. Antagoniste et/ou agoniste inverse du récepteur H₃ de l'histamine (B) tel que défini selon l'une quelconque des revendications 1 à 11 pour utilisation dans la prévention ou la correction des effets indésirables d'un traitement psychiatrique par un antipsychotique ou un antidépresseur (A) présentant un effet antagoniste du récepteur de l'histamine H₁ et choisi parmi l'olanzapine, la clozapine, la rispéridone, la quiétapine, la mirtazapine, l'aripiprazole, la paroxétine et l'amitryptyline, sur la prise de poids occasionnée ou susceptible d'être occasionnée par ledit traitement pour administration en complément d'un tel traitement.

16. Combinaison d'un composé (A) et d'un composé (B) tels que définis selon l'une quelconque des revendications 1 à 11 pour utilisation dans la prévention et/ou le traitement d'une pathologie choisie parmi : la schizophrénie, la dépression, la psychose, les désordres mentaux, la manie, les troubles affectifs bipolaires.

## Claims

1. Novel medicament comprising, in a pharmaceutically acceptable vehicle, an antipsychotic or an antidepressant (A), which, on its own, has an undesirable effect of a gain in body weight or sedation, and an antagonist and/or inverse agonist (B) of the histamine H₃ receptor, said antipsychotic or antidepressant being present in the medicament in a therapeutically effective amount for the antipsychotic or antidepressant effect sought, and the antagonist and/or inverse agonist of the histamine H₃ receptor being present in a therapeutically effective amount for ensuring at least one of the following three effects: suppression or at least limitation of the undesirable effect of weight gain, suppression or limitation of the undesirable effect on alertness, increase in the procognitive effect of the treatment, such that:
- agent (A) is selected from the agents having an antagonist effect on the histamine H₁ receptor, selected from olanzapine, clozapine, risperidone, quetiapine, mirtazapine, aripiprazole; and
- agent (B) corresponds to the formula (II) 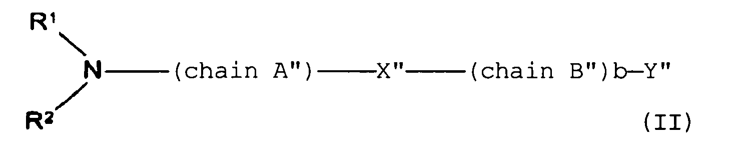 in which:
b = 0 or 1
i) R¹ and R² are identical or different and each independently represents
- a C₁-C₆ alkyl or a cycloalkyl
or taken together with the nitrogen atom to which they are fixed
- a saturated nitrogen-containing ring 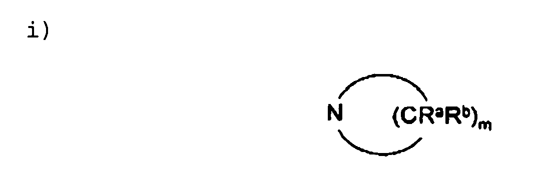 in which m is from 2 to 8 or
a non-aromatic unsaturated nitrogen-containing ring 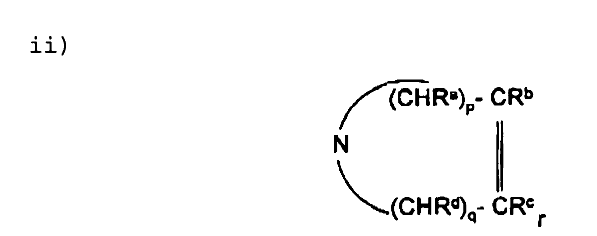 in which p and q are independently from 0 to 3 and r is from 0 to 4, provided that p and q are not simultaneously 0 and that 2 ≤ p+q+r ≤ 8,
R^{a-d} being independently a hydrogen atom or a C₁-C₆ alkyl group, cycloalkyl or carboalkoxy or
- a morpholino group or
- an N-substituted piperazino group 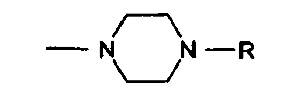 R being a C₁-C₆ alkyl group, cycloalkyl, carboalkoxy, aryl, arylalkyl, alkanoyl or an aroyl group,
ii) the chain A" is selected from a linear, branched, saturated or unsaturated -(-CH₂)_{n"}- group in which n" is a whole number from 1 to 8; a linear or branched alkenylene group having 1 to 8 carbon atoms; and a linear or branched alkynylene group having 1 to 4 carbon atoms;
iii) the group X" is selected from -OCONH-, OCON(alkyl)-, -OCON(alkene)-, -OCO-, -OCOSNH-, -CH₂-, -O-, -OCH₂CO-, -S-, -CO-, -CS-, an amine or a saturated or unsaturated alkyl;
iv) the chain B" is selected from the linear or branched, saturated or unsaturated alkylenes having 1 to 8 carbon atoms; and -(CH₂)_{n"}(heteroatom)- where the heteroatom is preferably an oxygen or sulfur atom; n" being a whole number from 1 to 5; and
v) the group Y" represents a phenyl group which is unsubstituted or mono- or polysubstituted by one or more identical or different substituents selected from the halogen atoms, OCF₃, CHO, CF₃, SO₂N(alkyl)₂ such as SO₂N(CH₃)₂, NO₂, S(aryl), SCH₂(phenyl), a linear or branched alkene, a linear or branched alkyne optionally substituted by a trialkylsilyl radical, -O(alkyl), -O(aryl), -CH₂CN, a ketone, an aldehyde, a sulfone, an acetal, an alcohol, a C₁-C₆ alkyl, -CH=CH-CHO, -C(alkyl)=N-OH, -C(alkyl)=N-O(alkyl) and other ketone derivatives, -CH=NOH, -CH-NO(alkyl) and other aldehyde derivatives, -C(alkyl)=NH-CONH₂, and O-phenyl or the -OCH₂ (phenyl) group, -C(cycloalkyl)=NOH, -C(cycloalkyl)=NO(alkyl); an optionally substituted heterocycle, a cycloalkyl; a bicyclic group and preferably a norbornyl group; a phenyl ring fused to a heterocycle comprising a nitrogen heteroatom or to a carbocycle or to a heterocycle having a ketone function; a linear or branched C₁-C₆ alkyl; a linear or branched alkyne having 1 to 8 carbon atoms and in particular 1 to 5 carbon atoms; a linear or branched alkyl which is mono- or polysubstituted by phenyl groups which are unsubstituted or mono- or polysubstituted; a phenyl alkyl ketone in which the alkyl group is linear or branched or cyclic; a substituted or unsubstituted benzophenone; a substituted or unsubstituted, linear, branched or cyclic phenyl alcohol; a linear or branched alkene; a piperidyl group; a phenyl cycloalkyl group; a polycyclic group, in particular a fluorenyl group, a naphthyl or polyhydronaphthyl group or an indanyl group; a phenol group; a ketone or a ketone derivative; a diphenyl group, a phenoxyphenyl group; a benzyloxyphenyl group, -CN, -alkyl, -aryl, -alkylCOalkyl, - COOalkyl, -COalkyl, -COaryl, -COaralkyl, -COcycloalkyl, -OH, -alkyl(OH), -alkyl(Oalkyl), -NHCOalkyl, -NH₂,
or its pharmaceutically acceptable salts, hydrates, or hydrated salts, or the polymorphic crystalline structures of these compounds or their optical isomers, racemates, diastereoisomers or enantiomers.

2. Medicament according to claim 1 wherein said antipsychotic or antidepressant (A) is olanzapine.

3. Medicament according to claim 1 or 2 such that (B) has the formula (IIa) 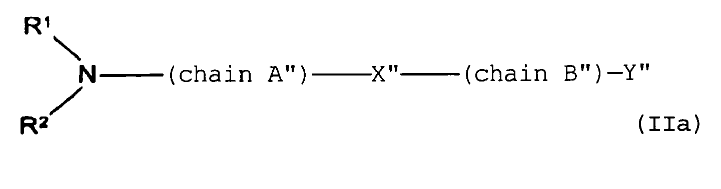 in which:
R¹ and R² are identical or different and each independently represents
- a saturated nitrogen-containing ring
i) 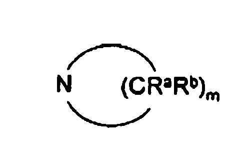 in which m is between 2 and 8,
R^{a-b} being selected independently from a hydrogen atom or a C₁-C₄ alkyl radical, and
ii) the chain A" is selected from a linear alkyl - -(CH₂)_{n"}-group in which n" is 3;
iii) the group X" is -O-;
iv) the chain B" is a linear alkyl group having 3 carbon atoms; and
v) the group Y" represents a phenyl group which is unsubstituted or mono- or polysubstituted by one or more identical or different substituents selected from the halogen atoms, OCF₃ , CHO, CF₃ , SO₂N(alkyl)₂ such as SO₂N (CH₃)₂, NO₂, S(aryl), SCH₂ (phenyl) , -alkylCOalkyl, -COOalkyl, -COalkyl, -COaryl, -COaralkyl, -COcycloalkyl, -alkyl(OH), -alkyl(Oalkyl), -Oaryl, -NHCOalkyl, -Oalkyl, -C(alkyl)=N-OH, -C(alkyl)=N-O(alkyl), -NO₂, -NH₂, a linear or branched alkene, a linear or branched alkyne optionally substituted by a trialkylsilyl radical, -O(alkyl), -O(aryl), -CH₂CN, a ketone, an aldehyde, a sulfone, an acetal, an alcohol, a C₁-C₄ alkyl, -CH=CH-CHO, -C(alkyl)=N-OH, -C(alkyl)=N-O(alkyl) and other ketone derivatives, -CH=NOH, -CH=NO(alkyl) and other aldehyde derivatives, -C(alkyl)=NH-NH-CONH₂, O-phenyl or -OCH₂(phenyl), -C(cycloalkyl)=NOH, -C(cycloalkyl)=NO(alkyl);
or its pharmaceutically acceptable salts, hydrates, or hydrated salts, or the polymorphic crystalline structures of these compounds or their optical isomers, racemates, diastereoisomers or enantiomers.

4. Medicament according to one of the preceding claims, wherein the grouping Y" is a phenyl group substituted by a halogen atom.

5. Medicament according to one of the preceding claims, wherein said compound (B) is 3-(4-chlorophenyl)propyl-3-piperidinopropyl ether (BF2649) or its pharmaceutically acceptable salts, hydrates, or hydrated salts, or the polymorphic crystalline structures of these compounds or their optical isomers, racemates, diastereoisomers or enantiomers.

6. Medicament according to one of the preceding claims, wherein the proportions of compound (A) relative to compound (B) are from 5 to 100 mg of compound (B) for 0.5 to 50 mg of compound (A).

7. Medicament according to one of the preceding claims which is suitable for oral administration.

8. Medicament according to claim 7 in the form of tablets, capsules, powder or a drinkable preparation.

9. Medicament according to one of the preceding claims, in particular in the form of a tablet, capsule or drinkable preparation combining 5 to 80 mg of compound (BF2649) with 3 to 20 mg of olanzapine.

10. Medicament according to one of the preceding claims, in particular in the form of a tablet, capsule or drinkable preparation combining 5 to 80 mg of compound (BF2649) with 0.5 to 10 mg of risperidone.

11. Medicament according to one of the preceding claims, in particular in the form of a tablet, capsule or drinkable preparation combining 5 to 80 mg of compound (BF2649) with 10 to 30 mg of aripiprazole.

12. Antagonist and/or inverse agonist of the histamine H₃ receptor (B) as defined in one of the preceding claims for use in the prevention or correction of the undesirable effects of a psychiatric treatment with an antipsychotic or an antidepressant (A) according to one of the preceding claims on alertness which is caused or may be caused by said treatment or in order to potentiate the therapeutic effects of said treatment on the cognitive sphere, for administration in addition to such a treatment.

13. Antagonist and/or inverse agonist of the histamine H₃ receptor (B) as defined in one of claims 1 to 11 for use in the prevention or correction of epilepsy and/or convulsions which are caused or may be caused by a psychiatric treatment with an antipsychotic or an antidepressant (A) as defined in one of claims 1 to 11 for administration in addition to such a treatment.

14. Antagonist and/or inverse agonist of the histamine H₃ receptor (B) as defined in one of claims 1 to 11 for use in the potentiation of the therapeutic effect on the cognitive sphere of a psychiatric treatment with an antipsychotic or an antidepressant (A) as defined in one of claims 1 to 11 for administration in addition to such a treatment.

15. Antagonist and/or inverse agonist of the histamine H₃ receptor (B) as defined in one of claims 1 to 11 for use in the prevention or correction of the undesirable effects of a psychiatric treatment with an antipsychotic or an antidepressant (A) having an antagonist effect on the histamine H₁ receptor and selected from olanzapine, clozapine, risperidone, quetiapine, mirtazapine, aripiprazole, paroxetine and amitryptyline, on the weight gain which is caused or may be caused by said treatment for administration in addition to such a treatment.

16. Combination of a compound (A) and a compound (B) as defined in one of claims 1 to 11 for use in the prevention and/or treatment of a pathology selected from: schizophrenia, depression, psychosis, mental disorders, mania, bipolar affective disorders.

## Patentansprüche

1. Neues Arzneimittel, umfassend in einem pharmazeutisch annehmbaren Träger, ein Antipsychotikum oder ein Antidepressivum (A), welches allein einen unerwünschten Effekt der Zunahme des Körpergewichts oder der Sedierung aufweist, und einen Antagonisten und/oder inversen Agonisten (B) des Rezeptors H₃ von Histamin, wobei das Antipsychotikum oder das Antidepressivum in dem Arzneimittel in einer therapeutisch wirksamen Menge für den angestrebten antipsychotischen oder antidepressiven Effekt vorhanden ist, und der Antagonist und/oder inverse Agonist des Rezeptors H₃ von Histamin in einer therapeutisch wirksamen Menge vorhanden ist zur Sicherstellung mindestens eines der folgenden Effekte: Unterdrückung oder mindestens Begrenzung des unerwünschten Effekts der Gewichtszunahme, Unterdrückung oder Begrenzung des unerwünschten Effekts auf die Wachsamkeit und Steigerung des pro-kognitiven Behandlungseffekts, wobei:
- der Wirkstoff (A) aus Mitteln ausgewählt ist, die einen antagonistischen Effekt auf den Rezeptor H₁ von Histamin ausüben, ausgewählt aus Olanzapin, Clozapin, Risperidon, Quietapin, Mirtazapin und Aripiprazol; und
- der Wirkstoff (B) der Formel (II) entspricht 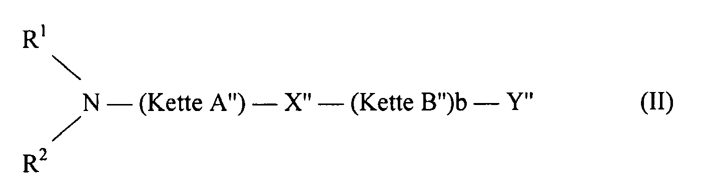 in der:
b = 0 oder 1
i) R¹ und R², die identisch oder verschieden sind, jeweils unabhängig voneinander
- C1-C6-Alkyl oder Cycloalkyl,
oder gemeinsam mit dem Stickstoffatom, an das sie gebunden sind,
- einen gesättigten stickstoffhaltigen Ring 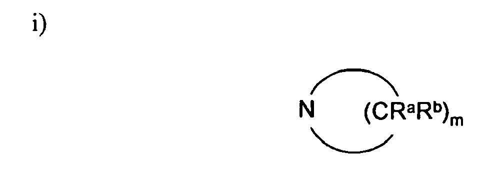 worin m 2 bis 8 bedeutet, oder
einen ungesättigten, nichtaromatischen stickstoffhaltigen Ring 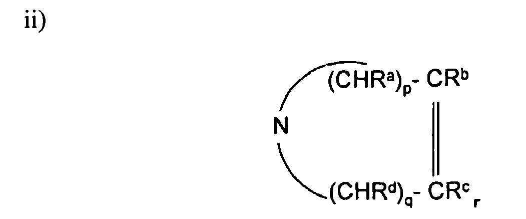 worin p und q unabhängig voneinander 0 bis 3 und r 0 bis 4 bedeuten, mit der Maßgabe, dass p und q nicht gleichzeitig 0 darstellen und 2 ≤ p + q + r ≤ 8 gilt, R^{a-d} unabhängig voneinander ein Wasserstoffatom oder eine C1-C6-Alkylgruppe, Cycloalkylgruppe oder Carboxyalkylgruppe oder
- eine Morpholinogruppe oder
- eine N-substituierte Piperazinogruppe 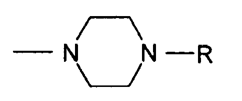 worin R eine C1-C6-Alkylgruppe, Cycloalkylgruppe, Carboxyalkylgruppe, Aryl-gruppe, Arylalkylgruppe, Alcanoylgruppe oder Aroylgruppe darstellt, bedeuten,
ii) die Kette A" aus einer geradkettigen verzweigten, gesättigten oder ungesättigten Alkylengruppe -(CH₂)_{n"}-, worin n" eine ganze Zahl mit einem Wert von 1 bis 8 darstellt; einer geradkettigen oder verzweigten Alkenylenkette mit 1 bis 8 Kohlenstoffatomen; und einer geradkettigen oder verzweigten Alkinylengruppe mit 1 bis 4 Kohlenstoffatomen ausgewählt ist;
iii) die Gruppe X" aus -OCONH-, OCON(Alkyl)-, -OCON(Alken)-, -OCO-, -OCOSNH-, -CH₂-, -O-, -OCH₂CO-, -S-, -CO-, -CS-, einer Aminogruppe oder einer gesättigten oder ungesättigten Alkylgruppe ausgewählt ist;
(iv) die Kette B" aus geradkettigen, verzweigten, gesättigten oder ungesättigten Alkylengruppen mit 1 bis 8 Kohlenstoffatomen; und Gruppen -(CH₂)_{n"}(Heteroatom)- ausgewählt ist, worin das Heteroatom vorzugsweise ein Sauerstoffatom oder Schwefelatom ist; und n" eine ganze Zahl mit einem Wert von 1 bis 5 darstellt; und
(v) die Gruppe Y" bedeutet eine Phenylgruppe, die nichtsubstituiert, monosubstituiert oder polysubstituiert ist durch einen oder mehrere, identische oder verschiedenartige Substituenten ausgewählt aus Halogenatomen, OCF₃, CHO, CF₃, SO₂N(Alkyl)₂, wie SO₂N(CH₃)₂, NO₂, S(Aryl), SCH₂(Phenyl), geradkettigem oder verzweigtem Alken, geradkettigem oder verzweigtem Alkin, gegebenenfalls substituiert durch einen Trialkylsilylrest, -O(Alkyl), -O(Aryl), -CH₂CN, ein Keton, einen Aldehyd, ein Sulfon, ein Acetal, einen Alkohol, C₁-C₆-Alkyl, -CH=CH-CHO, -C(Alkyl)=N-OH, -C(Alkyl)=N-O(alkyl) und andere Ketonderivate, -CH=NOH, -CH=NO(Alkyl) und andere Aldehydderivate, -C(Alkyl)=NH-CONH₂ und O-Phenyl oder die Gruppe -OCH₂(Phenyl), -C(Cycloalkyl)=NOH, -C(Cycloalkyl)=N-O(alkyl); einen gegebenenfalls substituierten Heterocyclus, eine Cycloalkylgruppe; eine bicyclische Gruppe und vorzugsweise eine Norbomylgruppe; einen Phenylkem, der an einen ein Stickstoffheteroatom umfassenden Heterocyclus oder einen Carbocyclus oder einen eine Ketonfunktion aufweisenden Heterocyclus kondensiert ist; eine geradkettige oder verzweigte C₁-C₆-Alkylgruppe; eine geradkettige oder verzweigte Alkingruppe, die 1 bis 8 Kohlenstoffatome und insbesondere 1 bis 5 Kohlenstoffatome umfasst; eine geradkettige oder verzweigte Alkylgruppe, die durch nichtsubstituierte oder mono- oder polysubstituierte Phenylgruppen mono- oder polysubstituiert ist; eine Phenylalkylketongruppe, bei der die Alkylgruppe geradkettig oder verzweigt oder cyclisch ist; ein substituiertes oder nichtsubstituiertes Benzophenon; einen substituierten oder nichtsubstituierten, geradkettigen, verzweigten oder cyclischen Phenylalkohol; ein geradkettiges oder verzweigtes Alken; eine Piperidylgruppe; eine Phenylcycloalkylgruppe; eine polycyclische Gruppe, insbesondere eine Fluorenylgruppe, eine Naphthylgruppe oder eine Polyhydronaphthylgruppe oder eine Indanylgruppe; eine Phenolgruppe; ein Keton oder ein Ketonderivat; eine Diphenylgruppe, eine Phenoxyphenylgruppe; eine Benzyloxyphenylgruppe, -CN, -Alkyl, -Aryl, -AlkylCOalkyl, -COOAlkyl, -COAlkyl, -COAryl, -COAralkyl, -COCycloalkyl, -OH, -Alkyl(OH), -Alkyl(Oalkyl), -NHCOAlkyl, -NH₂,
oder seine pharmazeutisch annehmbaren Salze, Hydrate oder hydratisierten Salze oder die polymorphen kristallinen Strukturen dieser Verbindungen oder deren optische Isomeren, Racemate, Diastereoisomeren oder Enantiomeren.

2. Arzneimittel nach Anspruch 1, worin das Antipsychotikum oder Antidepressivum (A) Olanzapin ist.

3. Arzneimittel nach Anspruch 1 oder 2, worin (B) der Formel (IIa) entspricht 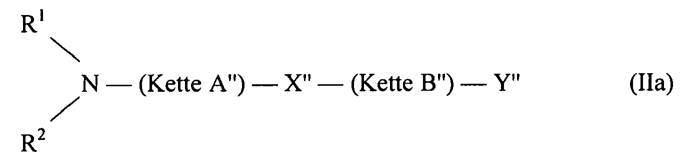 in der:
R¹ und R², die identisch oder verschieden sind, jeweils unabhängig voneinander
• einen gesättigten stickstoffhaltigen Cyclus
i) 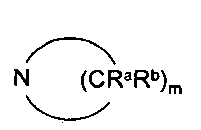 bedeuten, worin m zwischen 2 bis 8 liegt,
R^{a-b} unabhängig voneinander aus Wasserstoffatomen oder C1-C4-Alkylgruppen ausgewählt sind und
ii) die Kette A" aus einer linearen Alkylgruppe -CH₂)_{n"}-, worin n" 3 bedeutet, ausgewählt ist;
iii) die Gruppe X" -O- bedeutet;
(iv) die Kette B" eine geradkettige Alkylgruppe ist, die 3 Kohlenstoffatome aufweist; und
(v) die Gruppe Y" eine Phenylgruppe, die nichtsubstituiert, mono- oder polysubstituiert ist mit einem oder mehreren, identsichen oder unterschiedlichen Substituenten ausgewählt aus Halogenatomen, OCF₃, CHO, CF₃, SO₂N(Alkyl)₂, wie SO₂N(CH₃)₂, NO₂, S(Aryl), SCH₂(Phenyl), -AlkylCOalkyl, -COOAlkyl, -COAlkyl, -COAryl, -COAralkyl, -COCycloalkyl, -Alkyl(OH), -Alkyl(Oalkyl), -OAryl, -NHCOAlkyl, -OAlkyl, -C(Alkyl)=N-OH, -C(Alkyl)=N-O(alkyl), -NO₂, -NH₂, ein geradkettiges oder verzweigtes Alken, ein geradkettiges oder verzweigtes Alkin, das gegebenenfalls mit einer Trialkylsilylgruppe substituiert ist, -O(Alkyl), -O(Aryl), -CH₂CN, ein Keton, einen Aldehyd, ein Sulfon, ein Acetal, einen Alkohol, eine C1-C4-Alkylgruppe, -CH=CH-CHO, - C(Alkyl)=N-OH, -C(Alkyl)=N-O(alkyl) und andere Ketonderivate, -CH=NOH, - CH=NO(alkyl) und andere Aldehydderivate, -C(Alkyl)=NH-NH-CONH₂, O-Phenyl oder - OCH₂(Phenyl), -C-(Cycloalkyl)=NOH, -C(Cycloalkyl)=N-O(alkyl) bedeutet;
oder deren pharmazeutisch annehmbare Salze, Hydrate oder hydratisierten Salze oder die polymorphen kristallinen Strukturen dieser Verbindungen oder ihre optischen Isomere, Racemate, Diastereoisomere oder Enantiomere.

4. Arzneimittel nach einem der vorhergehenden Ansprüche, worin die Gruppe Y" eine durch ein Halogenatom substituierte Phenylgruppe ist.

5. Arzneimittel nach einem der vorhergehenden Ansprüche, worin die Verbindung (B) 3-(4-Chlorphenyl)-propyl-3-piperidinopropylether (BF2649) oder eines seiner pharmazeutisch annehmbaren Salze, Hydrate oder hydratisierten Salze oder die polymorphen kristallinen Strukturen dieser Verbindungen oder ihre optischen Isomeren, Racemate, Diastereoisomeren oder Enantiomeren ist.

6. Arzneimittel nach einem der vorhergehenden Ansprüche, worin die Anteile der Verbindung (A) bezogen auf die Verbindung (B) 5 bis 100 mg der Verbindung (B) pro 0,5 bis 50 mg der Verbindung (A) betragen.

7. Arzneimittel nach einem der vorhergehenden Ansprüche, die für die orale Verabreichung geeignet ist.

8. Arzneimittel nach Anspruch 7 in Form von Tabletten, Gelkapseln, eines Pulvers oder eines trinkbaren Präparats.

9. Arzneimittel nach einem der vorhergehenden Ansprüche, insbesondere in Form einer Tablette, Gelkapsel oder eines trinkbaren Präparats, umfassend 5 bis 80 mg der Verbindung (BF2649) und 3 bis 20 mg Olanzapin.

10. Arzneimittel nach einem der vorhergehenden Ansprüche, insbesondere in Form einer Tablette, Gelkapsel oder eines trinkbaren Präparats, umfassend 5 bis 80 mg der Verbindung (BF2649) und 0,5 bis 10 mg Risperidon.

11. Arzneimittel nach einem der vorhergehenden Ansprüche, insbesondere in Form einer Tablette, Gelkapsel oder eines trinkbaren Präparats, umfassend 5 bis 80 mg der Verbindung (BF2649) und 10 bis 30 mg Aripiprazol.

12. Antagonist und/oder inverser Agonist des Rezeptors H₃ von Histamin (B), wie er in einem der vorhergehenden Ansprüche definiert worden ist, zur Verwendung bei der Vorbeugung oder Verbesserung von unerwünschten Effekten einer psychiatrischen Behandlung mit einem Antipsychotikum oder einem Antidepressivum (A) nach einem der obigen Ansprüche auf die Wachsamkeit, die durch diese Behandlung beeinträchtigt oder möglicherweise beeinträchtigt ist, oder zur Verstärkung der therapeutischen Effekte der Behandlung auf den kognitiven Bereich, zur Verabreichung als Ergänzung einer solchen Behandlung.

13. Antagonist und/oder inverser Agonist des Rezeptors H₃ von Histamin (B), wie er in einem der Ansprüche 1 bis 11 definiert worden ist, zur Verwendung bei der Vorbeugung oder der Verhinderung der Epilepsie und/oder von Krämpfen, die durch eine psychiatrische Behandlung mit einem Antipsychotikum oder einem Antidepressivum (A), wie es in einem der Ansprüche 1 bis 11 definiert worden ist, bedingt sind oder bedingt werden können als Ergänzung einer solchen Behandlung.

14. Antagonist und/oder inverser Agonist des Rezeptors H₃ von Histamin (B), wie er in einem der Ansprüche 1 bis 11 definiert worden ist, zur Verwendung bei der Verstärkung des therapeutischen Effekts auf die kognitive Sphäre einer psychiatrischen Behandlung mit einem Antipsychotikum oder einem Antidepressivum (A), wie es in einem der Ansprüche 1 bis 11 definiert worden ist, zur Verabreichung als Ergänzung einer solchen Behandlung.

15. Antagonist und/oder inverser Agonist des Rezeptors H₃ von Histamin (B), wie er in einem der Ansprüche 1 bis 11 definiert worden ist, zur Verwendung bei der Vorbeugung oder der Verbesserung von unerwünschten Effekten bei einer psychiatrischen Behandlung mit einem Antipsychotikum oder einem Antidepressivum (A), das einen antagonistischen Effekt auf den Rezeptor des Histamins H₁ aufweist und aus Olanzapin, Clozapin, Risperidon, Quietapin, Mirtazapin, Aripiprazol, Paroxetin und Amitryptilin, auf die Gewichtszunahme, die durch diese Behandlung verursacht wird oder verursacht werden kann, zur Verabreichung als Ergänzung einer solchen Behandlung.

16. Kombination aus einer Verbindung (A) und einer Verbindung (B), wie sie in einem der Ansprüche 1 bis 11 definiert worden sind, zur Verwendung bei der Vorbeugung und/oder der Behandlung einer Erkrankung ausgewählt aus: Schizophrenie, Depression, Psychose, mentale Störungen, Manie, bipolaren affektiven Störungen.
